Europäisches Patentamt

(19) European Patent Office

Office européen des brevets

(11) Veröffentlichungsnummer: **0 021 050**
**B1**

(12) # EUROPÄISCHE PATENTSCHRIFT

(45) Veröffentlichungstag der Patentschrift:
27.07.83

(21) Anmeldenummer: 80102862.2

(22) Anmeldetag: 22.05.80

(51) Int. Cl.³: **C 12 N 9/96**, G 01 N 33/58

(54) Verfahren zur Gewinnung von immunologisch aktiven enzymmarkierten Konjugaten.

(30) Priorität: 07.06.79 DE 2923139

(43) Veröffentlichungstag der Anmeldung:
07.01.81 Patentblatt 81/1

(45) Bekanntmachung des Hinweises auf die Patenterteilung:
27.07.83 Patentblatt 83/30

(84) Benannte Vertragsstaaten:
AT BE CH DE FR GB IT LI LU NL SE

(56) Entgegenhaltungen:
DE-A-2 223 385
DE-A-2 430 357
JOURNAL OF IMMUNOLOGY, Band 116, Nr. 6, Juni 1976, Seiten 1554—1560, KANEFUSA KATO et al.: »Enzymelinked immunoassay: conjugation of the Fab' fragment of rabbit IgG with beta-D-Galactosidase from E. Coli and its use for immunoassay«
CHEMICAL ABSTRACTS, Band 90, Nr. 13, 26. März 1979, Seite 408, Nr. 101666k, Columbus, Ohio, USA, M. LANNER et al.: »Purification of enzyme-labeled conjugate by affinity chromatography«

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: Albert, Winfried, Dr., Moosstrasse 10,
D-8121 Pähl (DE)
Erfinder: Lenz, Helmut, Dr., Waldschmidtstrasse 7,
D-8132 Tutzing (DE)

(74) Vertreter: Weickmann, Heinrich, Dipl.-Ing. et al,
Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22,
D-8000 München 86 (DE)

CHEMICAL ABSTRACTS, Band 90, Nr. 13, 26. März 1979, Seite 408, Nr. 101667m, Columbus, Ohio, USA, R. VARRO et al.: »The use of affinity chromatography for purification of enzyme-antibody conjugates«

## Verfahren zur Gewinnung von immunologisch aktiven enzymmarkierten Konjugaten

Die Erfindung betrifft ein Verfahren zur Gewinnung von immunologisch aktiven enzymmarkierten Konjugaten, welche sich dazu eignen, bei immunologischen Nachweismethoden mit dem entsprechenden nichtmarkierten Antigen bzw. Hapten hinsichtlich der Bindung am Antikörper zu konkurrieren.

In neuerer Zeit hat sich neben dem länger bekannten Radio-Immuno-Assay (RIA) zunehmend der Enzym-Immuno-Assay (EIA) eingeführt, bei dem die radioaktive Markierung durch eine Enzymmarkierung ersetzt wird. Beim Enzym-Immuno-Assay konkurriert eine bekannte Menge enzymmarkierten Antigens bzw. Haptens mit der in der zu untersuchenden Probe vorhandenen unbekannten Menge des nichtmarkierten gesuchten Antigens bzw. Haptens um die Bindung am gemeinsamen Antikörper. Die Menge an antikörpergebundenem enzymmarkierten Antigen bzw. Hapten, zumeist als Konjugat bezeichnet, ist ein Maß für die Menge an gesuchtem Antigen bzw. Hapten.

Für diesen Enzym-Immuno-Assay werden Konjugate benötigt, welche immunologisch aktiv sind. Bei der Herstellung der Konjugate durch Markierung des Antigens bzw. Haptens werden jedoch zu einem erheblichen, zumeist überwiegenden Anteil immunologisch inaktive Konjugate gebildet. Derartige Gemische von immunologisch aktiven und inaktiven Konjugaten sind jedoch zumeist für den Enzym-Immuno-Assay wenig geeignet, da sie die Erreichung der an sich möglichen Sensitivität und Genauigkeit des Testsystems nicht zulassen. Beispielsweise können im Konjugat die immunologischen Eigenschaften des Immunreagens mit Enzymen verändert sein und hierdurch kann ein Verlust von antigenen Determinanten oder Bindungsstellen oder eine herabgesetzte Affinität für den Bindungspartner resultieren (vgl. Clin. Chem. Acta 81, 1 bis 40 [1977]).

Bekannt sind Verfahren zur Reinigung der rohen Konjugatgemische, die gewöhnlich nur niedrige Prozentsätze an Produkten der gesuchten Eigenschaften, wie hohe Immunreaktivität und hohe Enzymaktivität, aufweisen, welche auf Gelchromatographie oder Immunadsorption beruhen. Bei der Konjugatreinigung durch Immunadsorption treten übergroße Aktivitätsverluste auf, da Elutionsmittel fehlen, welche die Immunkomplexe spalten, ohne das Markierungsenzym zu inaktivieren bzw. die antigenen Eigenschaften zu verändern. Bei der Gelchromatographie werden unbefriedigende Trennergebnisse erhalten, da sich die hierfür ausschlaggebenden Eigenschaften der aktiven und der inaktiven Konjugate kaum unterscheiden.

Der Erfindung liegt daher die Aufgabe zugrunde, die oben geschilderten Nachteile zu beseitigen und ein Verfahren zu schaffen, welches in einfacher Weise eine befriedigende Trennung (Reinigung) der rohen Konjugatgemische aus immunologisch aktiven und inaktiven Konjugaten ermöglicht.

Gelöst wird diese Aufgabe erfindungsgemäß durch ein Verfahren zur Gewinnung von immunologisch aktiven enzymmarkierten Konjugaten aus einem durch kovalente Verknüpfung eines Enzyms mit einem Antigen bzw. Hapten erhaltenen Gemisch von immunologisch aktiven und inaktiven Konjugaten, welches dadurch gekennzeichnet ist, daß man eine Lösung dieses Gemisches mit einem unlöslichen Komplexbildner kontaktiert, welcher mit dem unmarkierten (freien) Antigen bzw. Hapten reversibel einen nichtimmunologischen Komplex zu bilden vermag, den unlöslichen Komplexbildner abtrennt und mit einem Desorbens für unmarkiertes (freies) Antigen bzw. Hapten eluiert.

Es hat sich überraschenderweise gezeigt, und hierauf beruht die Erfindung, daß die Haftfestigkeit bzw. Adsorption an derartigen Adsorbentien von der immunologischen Aktivität abhängig ist, derart, daß die immunologisch aktiven Konjugate stärker adsorbiert werden als die immunologisch nichtaktiven, so daß auch die immunologisch inaktiven oder weniger aktiven Konjugate durch das Desorbens leichter eluiert werden als die immunologisch aktiven Konjugate.

Als unlösliche Komplexbildner werden im Rahmen der Erfindung vorzugsweise solche verwendet, die durch Bindung an ein unlösliches Trägermaterial oder/und durch Vernetzung insolubilisiert sind.

Geeignete Komplexbildner für die jeweiligen Antigene oder Haptene sind in der Regel dem Fachmann bekannt. Handelt es sich beispielsweise bei dem Antigen um ein Protein ohne enzymatische Eigenschaften, beispielsweise steroidbindendes Protein, Serumalbumin oder TBG (thyroxinbindendes Blobulin), so wird man als Adsorbens die entsprechende Bindungskomponente, beispielsweise ein Steroid, Fettsäure, Thyroxin oder dergleichen, in insolubilisierter Form verwenden. Handelt es sich bei dem Antigen um ein Enzym, so wird als Komplexbildner zweckmäßig ein Coenzym, ein Substrat, ein Analogon davon oder ein Inhibitor desselben eingesetzt. Umgekehrt wird man bei Verwendung eines Hormons entweder ein hormonbindendes Protein oder eine niedermolekulare komplexbildende Substanz verwenden. Ist das Hapten ein Kohlehydrat, so eignet sich als Komplexbildner beispielsweise Lectin.

Methoden zur Insolubilisierung derartiger Komplexbildner an geeigneten Trägersubstanzen, wie z. B. Cellulose, Glas, Agarose und dergleichen, sind dem Fachmann bekannt und brauchen hier nicht näher beschrieben zu werden. Gleiches gilt für Verfahren zur Insolubilisierung durch Vernetzung mit polyfunktionellen Vernetzungsmitteln, beispielsweise Dialdehyden, wie Glutardialdehyd, Diepoxiden und dergleichen. Für diese Vernetzungsreaktionen zur Insolubilisierung gilt sinngemäß das gleiche, wie für die Insolubilisierung an einem Trägermaterial. Geeignete Insolubilisierungsmethoden sind z. B.

**0 021 050**

beschrieben in Colowick-Kaplan, Methods in Enzymology, 44, 11 bis 134, 263 bis 291 (1976).

Als Elutionsmittel verwendet man zweckmäßig entweder eine analoge Substanz oder den Komplexbildner selbst in gelöster Form. Auch Detergentien kommen in Betracht. Wichtig ist, daß ein Elutionsmittel gewählt wird, welches die Aktivität des Markierungsenzyms nicht wesentlich beeinträchtigt. Die Komplexbildung und die Elution werden stets in gepufferter Lösung mit einem für das Markierungsenzym geeigneten pH-Wert durchgeführt.

Typische Beispiele für Antigene und Haptene, die in enzymmarkierter Form erfindungsgemäß gereinigt werden können, sind TBG, Serumalbumin, Ligandin, steroidbindendes Protein, cyclo-AMPbindendes Protein, cyclo-GMP-bindendes Protein, Dehydrogenasen, Phospholiphase, Insulin, Kohlehydrate, Thyroxin, Trijodthyronin, Digoxin, Steroidhormone und Endorphine.

Typische Beispiele für geeignete Komplexbildner für die obengenannten Antigene und Haptene sind Thyroxin ($T_4$), Trijodthyronin ($T_3$), Fettsäure, Ligandin-Ligand, Steroid, Cyclo-AMP, Cyclo-GMP, NAD(H), Lipoprotein, Insulinrezeptor, die spezifischen Bindungsproteine der Haptene, wie Lecitin, TBG, Serumalbumin, Rinderserumalbumin, Charge Transfer-Ligand, steroidbindendes Globulin, Morphinrezeptor. Auch rein chemische Komplexbildner, wie z. B. Phenylbutylamin für $T_4$ und $T_3$, können verwendet werden.

In der nachstehenden Tabelle sind beispielsweise im Rahmen der Erfindung geeignete Antigene und Haptene mit den zugehörigen Komplexbildnern und Elutionsmitteln angeführt.

Tabelle

| Enzymmarkiertes Antigen oder Hapten | Komplexbildner, an Träger gebunden (unlöslich) | Elutionsmittel (gelöst) |
|---|---|---|
| 1 TBG | $T_4$-($T_3$-Sepharose) | $T_4$ oder ANS[1]) oder Salicylat |
| 2 Serumalbumin | Fettsäure | freie Fettsäure, Detergens |
| 3 Ligandin | Ligandin-Ligand | freier Ligandin-Ligand |
| 4 Steroidbindendes Protein | Steroid | freies Steroid |
| 5 c-AMP-bindendes Protein | c-AMP | gelöstes c-AMP |
| 6 c-GMP-bindendes Protein | c-GMP | gelöstes c-GMP |
| 7 Dehydrogenasen | NAD(H) | NAD(H) in Lösung |
| 8 Phospholipase | Lipoprotein | Desoxycholat |
| 9 Insulin | Insulinrezeptor | Insulin |
| 10 Kohlehydrat | Lectin | gelöster Zucker |
| 11 $T_4$ ($T_3$) Thyroxin (Trijodthyronin) | TBG | $T_4$, $T_3$ oder ANS[1]) |
| 12 $T_4$ ($T_3$) Thyroxin (Trijodthyronin) | Serumalbumin | Barbiturat, ANS[1]) |
| 13 $T_4$ ($T_3$) Thyroxin (Trijodthyronin) | Phenylbutylamin | nichtionisches Detergens |
| 14 Digoxin | Rinderserumalbumin | Barbiturat |
| 15 Digoxin | Charge Transfer Ligand | Ligand in Lösung |
| 16 Steroidhormon | steroidbindendes Globulin | Steroid in Lösung, Detergens |
| 17 Endorphin | Morphinrezeptor | Morphin bzw. Analoga |

[1]) ANS = Anilinonaphthalinsulfonsäure.

3

Das erfindungsgemäße Verfahren ermöglicht es, in einem einzigen einfachen Anreicherungsschritt den größten Teil des immunologisch inaktiven Konjugats zu entfernen und ein immunologisch aktives Konjugat zu gewinnen, welches eine überlegene Eignung für immunologische Nachweisverfahren aufweist.

Die folgenden Beispiele erläutern die Erfindung weiter.

## Beispiel 1

a)  Sepharose 4B wird nach üblicher Methode mit Bromcyan aktiviert und mit solcher Menge Rinderserumalbumin beladen, daß pro ml Sepharose ca. 5 bis 15 mg Albumin fixiert sind.

b)  Glucoseoxidase (EC. 1.1.3.4 aus Schimmelpilzen) wird mit 5- bis 10fach molarem Überschuß an aktiviertem Digoxinderivat (Aktivierung besteht in einer N-Hydroxysuccinimidester-Funktion, die kovalent an den Glycosid-Teil des Digoxinmoleküls gebunden ist) umgesetzt und nach ca. 24 bis 48 Stunden Reaktionszeit bei 4°C durch Dialyse vom überschüssigen Digoxinderivat abgetrennt. Das Primärkonjugat zeigt eine Immunreaktivität von ca. 6%, d.h. 6% der Glucoseoxidase im dialysierten Reaktionsgemisch binden an einen Immunadsorber, der im Überschuß Antidigoxin-Antikörper trägt.

c)  Das Primärkonjugat wird im Phosphatpuffer (z.B. 0,2 M, pH 7) über eine Säule mit einer Füllung von Rinderserumalbumin-Sepharose geschickt. Nach Auswaschen nicht bindender GOD-Aktivität mit Phosphatpuffer wird das interessierende Konjugat mit 0,12 M Barbiturat-Puffer, pH 8 (Bereich 7,5 bis 9) eluiert. Die Chromatographie wird so dimensioniert, daß ca. 20 ml Albuminsepharose/5 mg GOD im Primärkonjugat eingesetzt werden. Das gereinigte Konjugat ist zu ≥90% immunreaktiv im oben definierten Sinn.

## Beispiel 2

a)  Thyroxinbindendes Globulin (TBG), ein Glykoprotein, wird in Anlehnung an Nakane u. Kawaoi (H. Histochemistry, Cytochemistry, 22, 1084 bis 1091 [1974]) mit Perjodat aktiviert und im Bereich von pH 8 bis 10 mit 10- bis 30facher Gewichtsmenge $\beta$-Galactosidase (EC 3.2.1.23, aus E. coli) zur Kupplungsreaktion eingesetzt. Nach Reduktion mit Natriumborhydrid und Dialyse ergibt sich ein primäres Kupplungsprodukt, das je nach genauer Wahl der pH-Bedingungen, Umsetzungszeiten und Mengenverhältnisse zu 5 bis 20% immunreaktiv (im Sinne von % Bindung der im Kupplungsgemisch vorhandenen $\beta$-Galactosidase-Aktivität an ein Immunadsorbens mit Antikörper gegen TBG) ist.

b)  Thyroxin wird nach Kagedal und Kaellberg (Clin. Chim. Acta, 78, 103 [1977]) an Epoxy-Sepharose 6B (Pharmacia) in stabiler, kovalenter Bindung fixiert. Pro 1 mg TBG im Konjugatgemisch wird eine Säule mit ca. 3 ml Thyroxin-Sepharose gepackt und mit 10 bis 50 mM TRIS/0,1 M NaCl/10 mM $MgCl_2$-Puffer, pH 7,5 bis 8,5, äquilibriert. Auf diese Säule wird das rohe Konjugatgemisch (dialysiert gegen den gleichen Puffer) langsam aufgetragen und so lange mit Puffer nachgewaschen, bis im Ablauf keine $\beta$-Galactosidase-Aktivität mehr feststellbar ist.

Dann wird spezifisch gebundenes Konjugat mit Puffer eluiert, dem 2 bis 10 mM Anilinonaphthalinsulfonsäure (einem Agens, das spezifisch Thyroxin aus der Bindung mit TBG verdrängt) zugesetzt wird. Die $\beta$-Galactosidase-Aktivität im Eluat bindet zu ≥80% an Anti-TBG-Immunadsorbens.

## Patentansprüche

1. Verfahren zur Gewinnung von immunologisch aktiven enzymmarkierten Konjugaten aus einem durch kovalente Verknüpfung eines Enzyms mit einem Antigen bzw. Hapten erhaltenen Gemisch von immunologisch aktiven und inaktiven Konjugaten, dadurch gekennzeichnet, daß man eine Lösung dieses Gemisches mit einem unlöslichen Komplexbildner kontaktiert, welcher mit dem unmarkierten (freien) Antigen bzw. Hapten reversibel einen nichtimmunologischen Komplex zu bilden vermag, den unlöslichen Komplexbildner abtrennt und mit einem Desorbens für unmarkiertes (freies) Antigen bzw. Hapten eluiert.

2. Verfahren nach Anspruch 1, dadurch gekennzeichnet, daß ein durch Bindung an einen unlöslichen Träger insolubilisierter Komplexbildner verwendet wird.

3. Verfahren nach Anspruch 1, dadurch gekennzeichnet, daß ein durch Vernetzung insolubilisierter Komplexbildner verwendet wird.

4. Verfahren nach einem der vorhergehenden Ansprüche, dadurch gekennzeichnet, daß man als Desorbens gelösten Komplexbildner, ein Analogon davon oder ein Detergens verwendet.

5. Verfahren nach einem der vorhergehenden Ansprüche, dadurch gekennzeichnet, daß man bei einem Antigen, welches ein Protein ohne enzymatische Eigenschaften ist, als Komplexbildner dessen entsprechende Bindungskomponente, wie ein Steroid, eine Fettsäure, ein Hormon, verwendet.

6. Verfahren nach einem der Ansprüche 1 bis 4, dadurch gekennzeichnet, daß man bei einem Antigen, welches ein enzymatisch aktives Protein ist, als Komplexbildner ein Coenzym, Substrat, Substratanalogon oder einen Inhibitor verwendet.

7. Verfahren nach einem der Ansprüche 1 bis 4, dadurch gekennzeichnet, daß man bei einem Hapten mit Hormoncharakter als Komplexbildner ein hormonbildendes Protein oder eine niedermolekulare Substanz verwendet.


**Claims**

1. Process for obtaining immunologically-active, enzyme-marked conjugates from a mixture of immunologically-active and inactive conjugates obtained by covalent coupling of an enzyme with an antigen or hapten, characterised in that one contacts a solution of this mixture with an insoluble complex former which is able reversibly to form a nonimmunological complex with the unmarked (free) antigen or hapten, separates off the insoluble complex former and elutes with a desorbent for unmarked (free) antigen or hapten.

2. Process according to claim 1, characterised in that there is used a complex former insolubilised by bonding to an insoluble carrier.

3. Process according to claim 1, characterised in that there is used a complex former insolubilised by cross-linking.

4. Process according to one of the preceding claims, characterised in that as desorbent one uses dissolved complex former, an analogue thereof or a detergent.

5. Process according to one of the preceding claims, characterised in that in the case of an antigen which is a protein without enzymatic properties, as complex former one uses its corresponding binding components, such as a steroid, a fatty acid or a hormone.

6. Process according to one of claims 1 to 4, characterised in that in the case of an antigen which is an enzymatically-active protein, as complex former one uses a co-enzyme, substrate, substrate analogue or an inhibitor.

7. Process according to one of claims 1 to 4, characterised in that in the case of a hapten with hormone character, as complex former one uses a hormone-binding protein or a low molecular substance.


**Revendications**

1. Procédé d'extraction de conjugués immunologiquement actifs marqués par les enzymes à partir d'un mélange de conjugués immunologiquement actifs et inactifs obtenu par jonction covalente d'une enzyme avec un antigène ou un haptène, caractérisé en ce qu'on met en contact une solution de ce mélange avec un formateur de complexe insoluble qui est capable de former réversiblement un complexe non immunologique avec l'antigène ou l'haptène non marqué (libre), en ce qu'on sépare le formateur de complexe insoluble et en ce qu'on élue avec un désorbant pour l'antigène ou l'haptène non marqué (libre).

2. Procédé suivant la revendication 1, caractérisé en ce qu'on utilise un formateur de complexe insolubilisé par liaison à un support insoluble.

3. Procédé suivant la revendication 1, caractérisé en ce qu'on utilise un formateur de complexe insolubilisé par réticulation.

4. Procédé suivant l'une des revendications précédentes, caractérisé en ce qu'on utilise comme désorbant de formateur de complexe dissous un analogue de celui-ci ou un détergent.

5. Procédé suivant l'une des revendications précédentes, caractérisé en ce que dans le cas d'un antigène qui est une protéine sans propriétés enzymatiques, on utilise, comme formateur de complexe, son composant de liaison correspondant, tel qu'un stéroïde, un acide gras, une hormone.

6. Procédé suivant l'une des revendications 1 à 4, caractérisé en ce que dans le cas d'un antigène qui est une protéine enzymatiquement active, on utilise comme formateur de complexe une coenzyme, un substrat, un analogue de substrat ou un inhibiteur.

7. Procédé suivant l'une des revendications 1 à 4, caractérisé en ce que dans le cas d'un haptène ayant le caractère d'une hormone, on utilise comme formateur de complexe une protéine se liant aux hormones ou une substance de faible masse moléculaire.